Europäisches Patentamt

(19) European Patent Office

Office européen des brevets

(11) Veröffentlichungsnummer: **0 090 192**
**B1**

(12) **EUROPÄISCHE PATENTSCHRIFT**

(45) Veröffentlichungstag der Patentschrift:
**13.05.87**

(21) Anmeldenummer: **83102068.0**

(22) Anmeldetag: **03.03.83**

(51) Int. Cl.⁴: **G 01 N 33/86**

(54) Gerät zum Erfassen und Messen der Blutgerinnungszeit durch einen Impuls aufgrund der Lageveränderung einer Stahlkugel in einem mit Reagenz gefüllten Röhrchen.

(30) Priorität: **26.03.82 DE 3211191**

(43) Veröffentlichungstag der Anmeldung:
**05.10.83 Patentblatt 83/40**

(45) Bekanntmachung des Hinweises auf die Patenterteilung:
**13.05.87 Patentblatt 87/20**

(84) Benannte Vertragsstaaten:
**AT BE CH GB IT LI NL SE**

(56) Entgegenhaltungen:
**DE - A - 2 112 055**
**DE - A - 2 631 950**
**FR - A - 2 465 227**
**US - A - 3 635 678**

(73) Patentinhaber: **Behnk, Holger, Eppendorfer Weg 208, D-2000 Hamburg 20 (DE)**

(72) Erfinder: **Behnk, Holger, Eppendorfer Weg 208, D-2000 Hamburg 20 (DE)**

(74) Vertreter: **Schmidt-Bogatzky, Jürgen, Dr. Ing., Schlossmühlendamm 4, D-2100 Hamburg 90 (DE)**

**Beschreibung**

Die Erfindung betrifft ein Gerät zum Erfassen und Messen der Blutgerinnungszeit durch einen Impuls aufgrund der Lageveränderung einer Stahlkugel in einem mit Reagenz gefüllten Röhrchen.

Es ist bereits ein derartiges gattungsgemässes Gerät aus der FR-A-2 318 421 bekannt geworden. Dieses besteht aus einem schräggelagerten Röhrchen mit einer eingegebenen Stahlkugel. Das Röhrchen wird um seine Längsachse gedreht. Die Stahlkugel bleibt durch ihre Schwerkraft exakt an einer vorgegebenen Stelle, bis beim Einsetzen der Gerinnung durch Lageveränderung der Stahlkugel in einem magnetischen Sensor ein Impuls ausgelöst wird, der dann automatisch registriert wird. Der Nachteil dieses bekannten Gerätes besteht darin, dass es einer sehr präzisen Fertigung bedarf, da die Schräglage stets beibehalten werden muss. Dieses erfordert eine sorgfältig hergestellte Lagerung des Röhrchens, die z.B. bei Stosseinwirkungen auf das Gerät beeinträchtigt werden können. Erschütterungen können auch bewirken, dass die Kugel schon dann bewegt wird, wenn noch keine Gerinnung eingetreten ist.

Die Aufgabe der Erfindung besteht darin, das bekannte Gerät so weiter zu entwickeln, dass bei den verschiedensten Reagenzien eine sichere Ermittlung des Beginns der Blutgerinnung möglich ist.

Erfindungsgemäss erfolgt die Lösung der Aufgabe durch eine in dem Gerätgehäuse ausgebildete Halteeinrichtung zur senkrechten Halterung der Messküvette, unter der eine Magnetrühreinrichtung angeordnet ist, mittels derer die in der mit dem Reagenz gefüllten Messküvette befindliche Stahlkugel in eine umlaufende Bewegung versetzbar ist und einen der Stahlkugel zugeordneten, an der Aussenwand der Messküvette angeordneten Sensor einer Messeinrichtung, über den bei einer radialen Verlagerung der umlaufenden Stahlkugel nach innen eine Rechnereinheit steuerbar ist.

Durch die elektronische Abtastung ist die Blutgerinnungszeitermittlung unabhängig von Zwischentrübungen oder inhomogenen Reagenzien. Es sind daher Bestimmungen sowohl bei klaren Reagenzien wie auch bei Vollblut möglich. Durch Fremdlicht oder Erschütterungen können keine Störungen auftreten. Durch einen einfachen Kugelspender ist ein leichtes und rationelles Arbeiten möglich. Das Gerät weist eine sehr hohe Empfindlichkeit auch bei niedriger Fibrinogenkonzentration auf, wodurch auch ein sicherer Betrieb im pathologischen Bereich möglich ist.

Ausgestaltungen der Erfindung werden in den abhängigen Ansprüchen beschrieben.

Einzelheiten der Erfindung werden nachstehend anhand der in den Zeichnungen dargestellten Ausführungsbeispiele erläutert. Es zeigt

Fig. 1 eine Ausführungsform des erfindungsgemässen Gerätes mit einer Frequenzabtastung in einer schematischen Ansicht,

Fig. 2 eine weitere Ausführungsform des erfindungsgemässen Gerätes mit einer Durchlichtabtastung in einer schematischen Ansicht,

Fig. 3 eine weitere Ausführungsform des erfindungsgemässen Gerätes mit einer Mittelpunktsabtastung der Messküvette in einer schematischen Ansicht,

Fig. 4a die Kugelbahn in der Messküvette vor Eintritt der Gerinnung in der Draufsicht,

Fig. 4b die Kugelbahn in der Messküvette bei Eintritt der Gerinnung in der Draufsicht,

Fig. 5 eine grafische Darstellung der Reaktionskurve der Viskositätsänderungen bei Eintritt der Gerinnung,

Fig. 6 eine grafische Darstellung der Trübung der Flüssigkeit mit und ohne Kugel in der Messküvette.

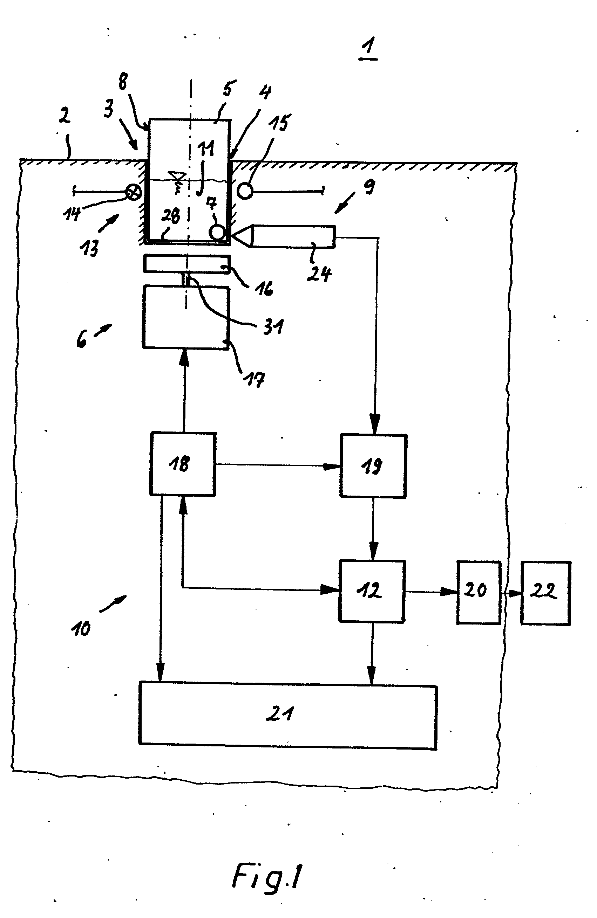

Das in Fig. 1 dargestellte Gerät 1 ist ein Beispiel für eine viskositäts-dynamische Messung. Es besteht auf einem in der Zeichnung nur angedeuteten Gehäuse 2, in dem eine sacklochartige Ausnehmung 4 als Halteeinrichtung für die Messküvette 5 ausgebildet ist. Die Messküvette 5 befindet sich in dieser Ausnehmung 4. Auf der Bodenplatte 28 ist eine Stahlkugel 7.

In der Ebene der Stahlkugel 7 befindet sich ein Sensor 9 einer Messeinrichtung 10. Unter der Bodenplatte 28 ist das Antriebsglied 16 der magnetischen Rühreinrichtung 6 angeordnet. Die magnetische Rühreinrichtung 6 besteht aus dem als Dauermagnet ausgebildeten Antriebsglied 16, das mit einem elektrischen Antriebsmotor 17 verbunden ist. Bei Rotation des Antriebsgliedes 16 wird die Stahlkugel 7 durch die einwirkende Magnetkraft in eine kreisförmige Bewegung versetzt, die aufgrund der Fliehkraft und Magnetkraft zunächst am Rande der Messküvette 5 stattfindet. Bei Eintritt der Gerinnung des in der Messküvette 5 befindlichen Blutes durch die Rührwirkung der Stahlkugel 7 wandert diese wegen Vergrösserung des Strömungswiderstandes auf einer Kurvenbahn nach innen zum Mittelpunkt der Bodenplatte 28. Diese Bewegung der Stahlkugel 7 wird von der Messeinrichtung 10 erfasst.

Die Messeinrichtung 10 weist eine Rechnereinheit 12 auf, die über einen Frequenzvergleicher mit dem Sensor 9 verbunden ist. Der Sensor 9 kann als Näherungsinitiator 24, z.B. als induktiver oder kapazitiver Näherungsinitiator, ausgebildet sein. Der Frequenzvergleicher 19 steht mit dem Frequenzgenerator 18 in Verbindung, welcher zur Gleichlaufsteuerung mit dem Antriebsmotor 17 verbunden ist. Die Istwertanzeige des Frequenzgenerators wird auf eine Anzeigeeinheit 21 abgebildet. Der Frequenzgenerator 18 ist auch mit der Rechnereinheit 12 verbunden. Diese steht mit der Anzeigeeinheit 21 sowie einem Datenspeicher 20 in Wirkverbindung, welcher an eine elektronische Datenverarbeitungseinrichtung 22 angeschlossen sein kann.

Der Start des Gerätes 1 kann durch mechanischen Starteingriff oder aber auch automatisch erfolgen. Hierzu kann beispielsweise eine lichtoptische Abtasteinrichtung 13 vorgesehen sein, die in einer Ebene oberhalb der Bodenplatte 28 angeordnet ist. Sie besteht aus einer Lichtquelle 14 und einem Lichtempfänger 15. Der Abstand der Ebene der lichtoptischen Abtasteinrichtung 13 von der Bodenplatte 28 entspricht der Mindestfüllhöhe der Messküvette 5. Sobald das in die Messküvette 5 einzufüllende Reagenz die Durchlässigkeit des von der Lichtquelle 14 ausgesandten Lichtes vermindert, ergeht ein Startimpuls an die Messeinrichtung 10 und den Antriebsmotor 17.

Die Abtastung der Umläufe der Stahlkugel 7 durch den Näherungsinitiator 24 erfolgt frequenzsynchron zu dem Antriebsmotor 17. Bei Aussetzen eines Impulses gibt die Rechnereinheit 12 einen Stopp-Steuerbefehl an den Antriebsmotor 17, wobei gleichzeitig eine Anzeige auf der Anzeigeeinheit 21 und eine Datenspeicherung in dem Datenspeicher 20 erfolgt.

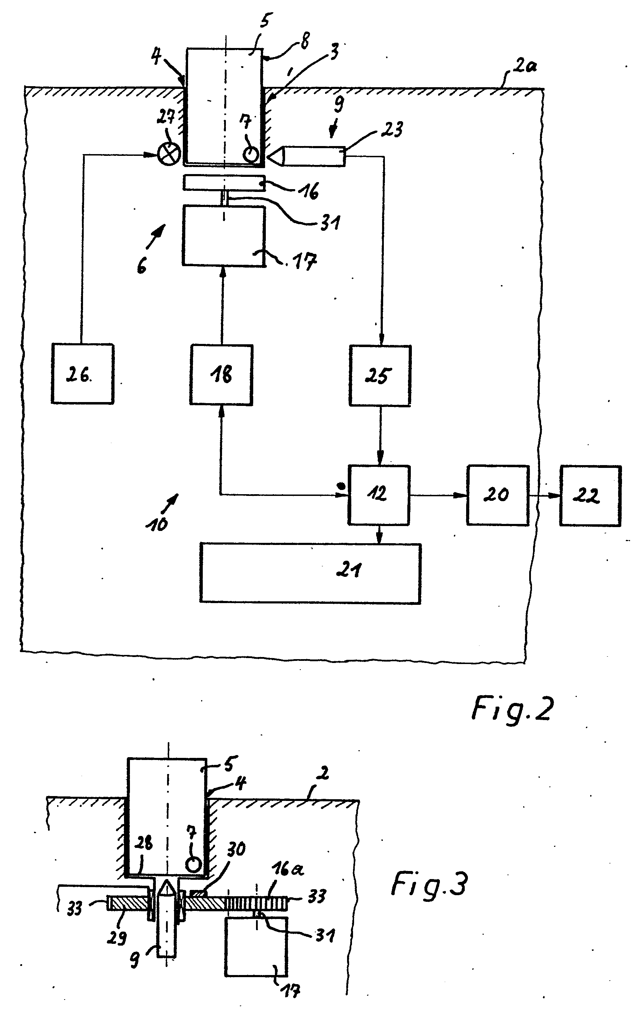

Wie in Fig. 2 dargestellt, kann auch ein Gerät 1a verwendet werden, bei dem der Sensor 9 als Reflex-Messwertaufnehmer 23 ausgebildet ist. Der Reflex-Messwertaufnehmer 23 kann als optischer oder akustischer Messwertaufnehmer ausgebildet sein. Als optischer Messwertaufnehmer wird die Reflexion von Licht auf der Oberfläche der umlaufenden Stahlkugel 7 wahrgenommen, während ein akustisch wirkender Messwertaufnehmer die Geräusche prüft, die die Stahlkugel 7 verursacht. Hierbei sind die vor Gerinnungseintritt entstehenden Geräusche anders strukturiert, als bei Gerinnungseintritt, bei dem die Stahlkugel 7 sich sofort zum Mittelpunkt bewegt und dort zum Stillstand kommt.

Dem Reflex-Messwertaufnehmer 23 ist an der gegenüberliegenden Seite der Messküvette 5 eine Lichtquelle 27 zugeordnet, die mit einem Lampenspannungsregler 26 verbunden ist. Der Lampenspannungsregler 26 dient zur Konstanthaltung der Spannung an der Lichtquelle 27. Der Reflex-Messwertaufnehmer 23 ist mit einem Fotoverstärker 25 verbunden, der wiederum mit der Rechnereinheit 12 in Wirkverbindung steht. Der Fotoverstärker 25 dient dazu, auch kleine Änderungen des Durchlichtes in einen für die Rechnereinheit 12 verwertbaren Impuls umzuwandeln.

Wie in Fig. 3 dargestellt, ist es auch möglich, als Messort für den Sensor 9 den Mittelpunkt der Bodenplatte 28 zu verwenden. Bei dieser Ausführung ergeht an die Messeinrichtung 10 ein Impuls, wenn die Stahlkugel 7 bei Eintritt der Gerinnung zum Mittelpunkt der Bodenplatte 28 wandert. Das Antriebsglied der Magnetrühreinrichtung ist als Kreisringscheibe 29 ausgebildet und koaxial zum Sensor 9 angeordnet. Auf der der Messküvette 5 zugewandten Fläche der Kreisringscheibe 29 befindet sich ein Dauermagnet 30. Randseitig ist an der Kreisringscheibe 29 eine Verzahnung ausgebildet, in die ein Zahnrad 33 eingreift, das auf der Antriebswelle 31 des Antriebmotors 17 gelagert ist.

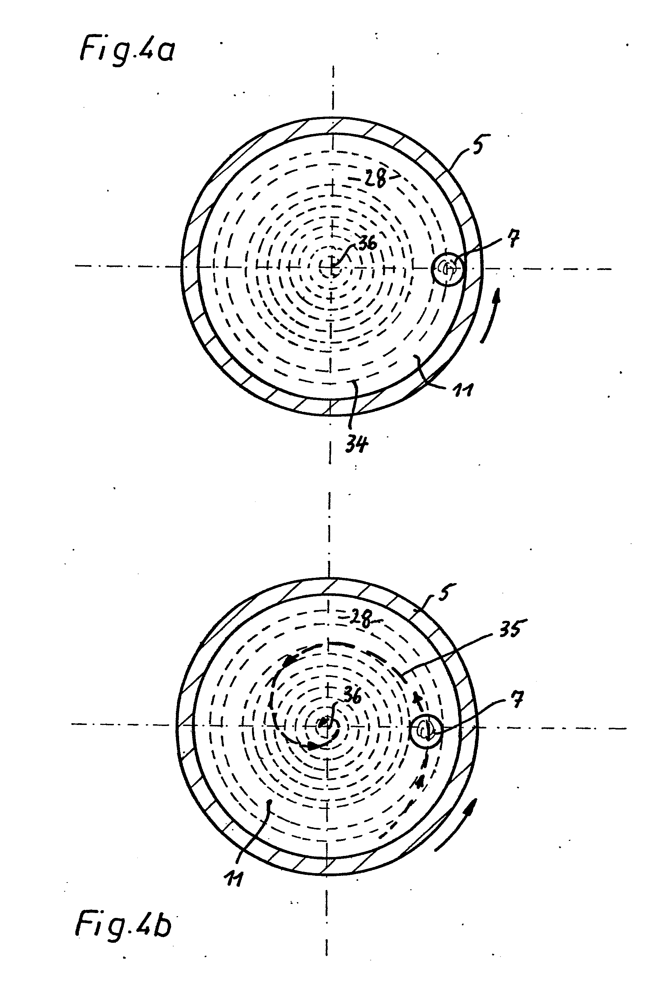

In Fig. 4a und 4b ist eine Messküvette 5 in der Draufsicht dargestellt und mit einem Reagenz 11 gefüllt. Die Stahlkugel 7 befindet sich auf der Bodenplatte 28 und liegt aufgrund der Zentrifugalkraft und Magnetkraft an der Wandung der Messküvette 5 zunächst an, wenn diese in Rotation versetzt wird. Sobald das Reagenz 11 durch Eintritt von Gerinnung inhomogen wird, verläuft die Bewegung der Stahlkugel 7 auf einer Kurvenbahn 35 bis zum Mittelpunkt 36 der Bodenplatte 28.

Während des Betriebszustandes gemäss Fig. 4a weist das zu messende Reagenz eine gleichmässige Viskosität auf. Die Strömungsgeschwindigkeit nimmt von innen nach aussen zu. Bei dem Betriebszustand gemäss Fig. 4b nimmt die Viskosität beim Einsetzen der Gerinnung am Rand der Messküvette 5, bedingt durch die höhere Strömungsgeschwindigkeit, schneller ab als zur Mitte. Hierdurch verläuft die Gerinnselbildung sehr schnell vom Rand der Messküvette zu deren Mitte. Ein zusätzlicher Effekt tritt dadurch auf, dass die Stahlkugel 7 gegen eine sich verfestigende Masse läuft und nur zum Mittelpunkt 36 ausweichen kann. Da das Magnetfeld von aussen zur Mitte der Bodenplatte 28 stark abnimmt, wird dieser Effekt untersützt.

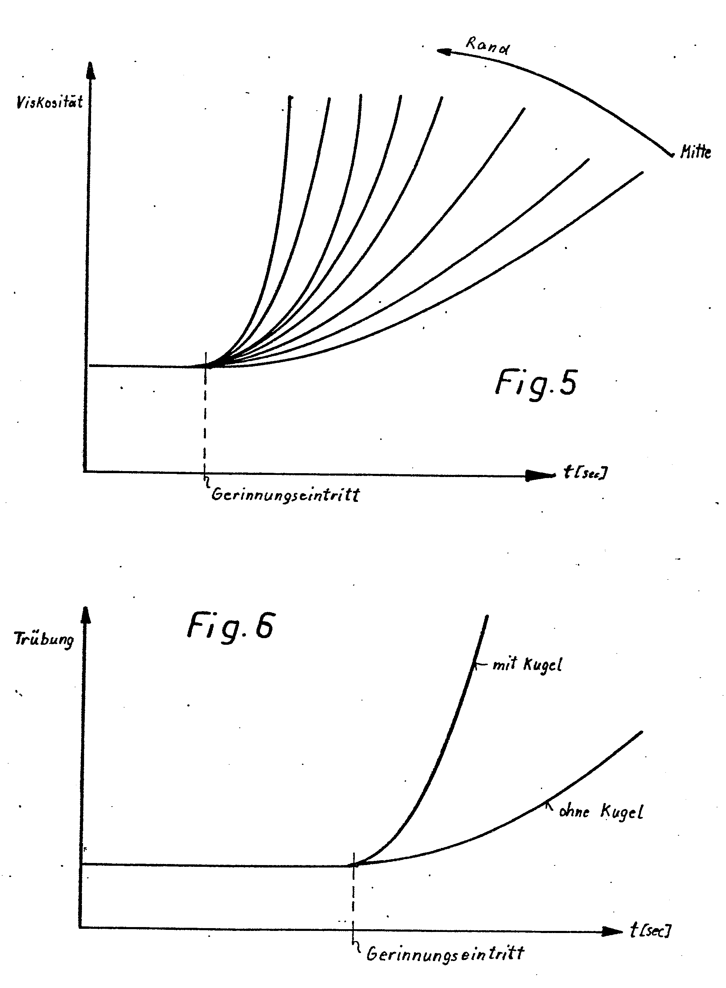

Die Viskositätsänderungen bei Eintritt der Gerinnung sind, bezogen auf den Abschnitt vom Rand der Messküvette 5 bis zu deren Mitte, in der Kurvenschar gemäss Fig. 5 dargestellt. Fig. 6 verdeutlicht die unterschiedlichen Trübungen bei Messdurchführungen mit und ohne Stahlkugel 7.

**Patentansprüche**

1. Gerät zum Erfassen und Messen der Blutgerinnungszeit durch einen Impuls aufgrund der Lageveränderung einer Stahlkugel in einem mit Reagenz gefüllten Röhrchen, gekennzeichnet durch eine in dem Gerätegehäuse (2) ausgebildete Halteeinrichtung (3) zur senkrechten Halterung der Messküvette (5), unter der eine Magnetrühreinrichtung (6) angeordnet ist, mittels derer die in der mit dem Reagenz gefüllten Messküvette (5) befindliche Stahlkugel (7) in eine umlaufende Bewegung versetzbar ist und einen der Stahlkugel (7) zugeordneten, an der Aussenwand (8) der Messküvette (5) angeordnete Sensor (9) einer Messeinrichtung (10), über den bei einer radialen Verlagerung der umlaufenden Stahlkugel (7) nach innen eine Rechnereinheit 12) steuerbar ist.

2. Gerät nach Anspruch 1, dadurch gekennzeichnet, dass in einer Ebene oberhalb der Stahlkugel (7) eine lichtoptische Abtasteinrichtung (13) angeordnet ist.

3. Gerät nach Anspruch 1, dadurch gekennzeichnet, dass die Magnetrühreinrichtung (6) in an sich bekannter Weise aus einem als Dauermagnet ausgebildeten Antriebsglied (16, 16a) besteht, dessen elektrischer Antriebsmotor (17) mit einem Frequenzgenerator (18) verbunden ist, der mit einem mit dem Sensor (9) verbundenen Frequenzvergleicher (19) in Wirkverbindung steht, der über eine Rechnereinheit (12) mit einem Datenspeicher (20) und/oder einer Anzeigeeinheit (21) verbunden ist.

4. Gerät nach Anspruch 3, dadurch gekennzeichnet, dass die Rechnereinheit (12) mit dem Frequenzgenerator (18) verbunden ist.

5. Gerät nach Anspruch 3, dadurch gekennzeichnet, dass der Datenspeicher (20) mit einer elektronischen Datenverarbeitungseinrichtung (22) in Wirkverbindung steht.

6. Gerät nach Anspruch 1 bis 5, dadurch gekennzeichnet, dass der Antriebsmotor (17) und die Messeinrichtung (10) durch einen Impuls der Abtasteinrichtung (13) einschaltbar ist.

7. Gerät nach Anspruch 1, dadurch gekennzeichnet, dass der Sensor (9) als optischer oder akustischer Reflex-Messwertaufnehmer (23) oder als induktiver oder kapazitiver Näherungsinitiator (24) ausgebildet ist.

8. Gerät nach Anspruch 1 und 2, 4 bis 7, dadurch gekennzeichnet, dass der als lichtoptischer Reflex-Messwertaufnehmer (23) ausgebildete Sensor (9) über einen Fotoverstärker (25) mit der Rechnereinheit (12) verbunden ist, die mit dem Datenspeicher (20) und/oder der Anzeigeeinheit (21) und dem elektrischen Antriebsmotor (17) der Magnetrühreinrichtung (6) verbunden ist.

9. Gerät nach Anspruch 8, dadurch gekennzeichnet, dass die Rechnereinheit (12) mit einem Lampenspannungsregler (26) verbunden ist, der mit der dem lichtoptischen Reflex-Messwertaufnehmer (23) zugeordneten Lichtquelle (27) in Wirkverbindung steht.

10. Gerät nach Anspruch 1 bis 7, dadurch gekennzeichnet, dass der Sensor (9) mittig unter der Bodenplatte (28) der Messküvette (5) angeordnet ist.

11. Gerät nach Anspruch 10, dadurch gekennzeichnet, dass das Antriebsglied (16a) als koaxial zum Sensor (9) angeordnete drehbar gelagerte Kreisringscheibe (29) ausgebildet ist, auf der der Messküvette (5) zugewandt ein Dauermagnet (30) befestigt ist und die mit der Antriebswelle (31) des Antriebsmotors (17) in Wirkverbindung steht.

12. Gerät nach Anspruch 11, dadurch gekennzeichnet, dass der Dauermagnet (30) die Kreisringscheibe (29) nur abschnittsweise bedeckend ausgebildet ist.

13. Gerät nach Anspruch 11, dadurch gekennzeichnet, dass auf der Antriebswelle (31) des Antriebsmotors (17) ein Zahnrad (32) gelagert ist, das mit einer randseitigen Verzahnung (33) an der Kreisringscheibe (29) in Wirkeingriff ist.

**Claims**

1. Device for detecting and measuring the time of coagulation of blood by an impulse through variation of the position of a steel ball in a tube filled with reagent characterized by an holding device (3) being modelled in the housing (2) of the device for a vertical holding of the measuring bulb (5), a magnet stirring device (6) being arranged below it, through which the steel ball (7) being in the measuring bulb (5) filled with reagent is put into revolving movement, and a sensor (9) of a measuring device (10) being arranged on the outside wall (8) of the measuring bulb (5) and being allocated to the steel ball (7), with which a computer device is controllable by a radial dislocation of the revolving steel ball (7) towards the inside.

2. Device according to claim 1, characterized in that a light-optical scanning device (13) being arranged in a plane above the steel ball (7).

3. Device according to claim 1, characterized in that, the magnet-stirring device (6) consists in well known matter of a driving member (16, 16a) being a permanent magnet, the electrical driving motor (17) being connected to a frequency generator (18), being in active connection to a sensor (9) being connected with a frequency comparator (19) which is connected with a data storage (20) and/or a display device (21) via a computer device (12).

4. Device according to claim 3, characterized in that the computer device (12) is connected with the frequency generator (18).

5. Device according to claim 3, characterized in that the data storage (20) is in active connection with a data processing device (22).

6. Device according to claim 1 to 5, characterized in that the driving motor (17) and the measuring device are able to cut in by an impulse of the scanning-device (13).

7. Device according to claim 1, characterized in that the sensor (9) is of the type of an optical oj acoustical reflective transducer (23) or an inductive or capacitive approach switch (24).

8. Device according to claim 1 and 2, 4, to 7, characterized in that the sensor (9) being of the type of a light-optical reflection transducer (23) is connected with the computer device (12) via a photoelectric amplifier (25), the computer device (12) is connected with a data storage (20) and/or the display device (21) and the electrical driving motor (17) of the magnetic stirring device (6).

9. Device according to claim 8, characterized in that the computer device (12) is connected with the light voltage regulator (26) which is connected to the light source (27) being allocated to the reflective transducer (23).

10. Device according to claim 1 to 7, characterized in that the sensor (9) is arranged centrically below the bottom plate (28) of the measuring bulb (5).

11. Device according to claim 10, characterized in that the driving member (16a) is of the type of a circular ring disc (29) being rotatable coaxial beared to the sensor (9) a permanent magnet (30) is fastened at the circular ring disc (29) being directed to the measuring ball (5), the circular ring device (29) is connected with the motor shaft (31) of the driving motor (17).

12. Device according to claim 11, characterized in that the permanent magnet (30) covers the circular ring disc (29) only sectional.

13. Device according to claim 11, characterized in that on the motor shaft (31) of the driving motor (17) a gearwheel (32) is stored which is connected with the toothing (33) modelled at the circular ring disc (29).

**Revendications**

1. Appareil pour détecter et mesurer le temps de coagulation du sang, par une impulsion en raison de la variation de position d'une bille d'acier dans un petit tube rempli de réactif, caractérisé par un dispositif de maintien (3) constitué dans le boîtier (2) de l'appareil en vue de maintenir verticalement la cuvette de mesure (5) sous laquelle est monté un dispositif (6) d'agitateur magnétique, au moyen duquel la bille d'acier (7) qui se trouve dans la cuvette de mesure (5) remplie de réactif peut être mise en mouvement circulaire, et caractérisé par un détecteur (9) d'un dispositif de mesure (10), détecteur monté sur la paroi extérieure (8) de la cuvette de mesure (5) et associé à la bille d'acier (7), et par l'intermédiaire duquel une unité calculatrice (12) est susceptible d'être commandée lors d'un déplacement radial de la bille d'acier tournante (7) vers l'intérieur.

2. Appareil selon la revendication 1, caractérisé en ce qu'un dispositif d'exploration (13) optique-lumineux est monté dans un plan au-dessus de la bille d'acier (7).

3. Appareil selon la revendication 1, caractérisé en ce que le dispositif (6) d'agitateur magnétique consiste, de manière connue en soi, en un organe d'entraînement (16, 16a) constitué sous la forme d'aimant permanent, et dont le moteur de commande électrique (17) est relié à un générateur de fréquence (18) qui est en liaison active avec un comparateur de fréquence (19) relié au détecteur (9) et relié également, par l'intermédiaire d'une unité calculatrice (12), à une mémoire de données (20) et/ou à une unité indicatrice (21).

4. Appareil selon la revendication 3, caractérisé en ce que l'unité calculatrice (12) est reliée au générateur de fréquence (18).

5. Appareil selon la revendication 3, caractérisé en ce que la mémoire de données (20) est en liaison active avec un dispositif électronique (22) de traitement de données.

6. Appareil selon les revendications 1 à 5, caractérisé en ce que le moteur de commande (17) et le dispositif de mesure (10) peuvent être mis en circuit sous l'effet d'une impulsion du dispositif d'exploration (13).

7. Appareil selon la revendication 1, caractérisée en ce que le détecteur (9) est constitué sous la forme de récepteur de valeur de mesure réflexe (23) ou sous la forme d'initiateur d'approximation (24) inductif ou capacitif.

8. Appareil selon les revendications 1 et 2, 4 à 7, caractérisé en ce que le détecteur (9) constitué sous la forme de récepteur de valeur de mesure réflexe (23) optique-lumineux, est relié, par l'intermédiaire d'un photo-amplificateur (25), à l'unité calculatrice (12), qui est reliée à la mémoire de données (20) et/ou l'unité indicatrice (21) et avec le moteur de commande électrique (17) du dispositif (6) d'agitateur magnétique.

9. Appareil selon la revendication 8, caractérisé en ce que l'unité calculatrice (12) est reliée à un régulateur (26) de tension de lampe, qui est en liaison active avec la source de lumière (27) associée au récepteur de valeur de mesure réflexe (23) optique-lumineux.

10. Appareil selon les revendications 1 à 7, caractérisé en ce que le détecteur (9) est disposé centralement au-dessous de la plaque de fond (28) de la cuvette de mesure (5).

11. Appareil selon la revendication 10, caractérisé en ce que l'organe d'entraînement (16a) est constitué sous la forme de disque (29) en anneau de cercle, monté de façon à pouvoir tourner, en étant disposé coaxialement au détecteur (9), disque sur lequel est fixé un aimant permanent (30) situé en regard de la cuvette de mesure (5) et qui est en liaison active avec l'arbre d'entraînement (31) du moteur de commande (17).

12. Appareil selon la revendication 11, caractérisé en ce que l'aimant permanent (30) est constitué en ne recouvrant que par tronçons le disque (29) en anneau de cercle.

13. Appareil selon la revendication 11, caractérisé en ce que, sur l'arbre d'entraînement (31) du moteur de commande (17) est montée une roue dentée (32), qui est en prise active avec une denture (33) prévue du côté du bord sur le disque (29) en anneau de cercle.

Fig.1

Fig.2

Fig.3

Fig.4a

Fig.4b

Fig.5

*Viskosität* (y-axis), *t [sec]* (x-axis), *Gerinnungseintritt*, *Rand*, *Mitte*

Fig.6

*Trübung* (y-axis), *t [sec]* (x-axis), *Gerinnungseintritt*, *mit Kugel*, *ohne Kugel*